# EUROPEAN PATENT APPLICATION

(11) **EP 3 932 416 A2**
(43) Date of publication of application: **05.01.2022**
(21) Application number: 20762101.2
(22) Date of filing: 24.02.2020
(51) Int. Cl.: A61K 35/747, A61P 3/00, A23L 33/135, C12N 1/20, A61P 19/00

(54) **COMPOSITION FOR IMPROVING, PREVENTING, OR TREATING BONE DISEASES OR METABOLIC DISEASES, INCLUDING NOVEL LACTOBACILLUS SAKEI CVL-001 STRAIN AND CULTURE MEDIUM THEREOF**

(30) Priority: 26.02.2019 KR 20190022670; 26.02.2019 KR 20190022671; 03.02.2020 KR 20200012763
(71) Applicant: Industry Foundation of Chonnam National University, Gwangju 61186 (KR)
(72) Inventor: PARK, Jong Hwan, Gwangju 61746 (KR); CHOI, Joo Hee, Suncheon-si, Jeollanam-do 57936 (KR); JUNG, Do Hyeon, Gwangju 61684 (KR)
(74) Representative: Plougmann Vingtoft a/s
(86) International application number: PCT/KR2020/002612
(87) International publication number: WO 2020/175869

(57) **Abstract**

The present invention relates to a Lactobacillus sakei CVL-001 strain and a method for isolating same, wherein the strain is lactic acid bacteria which can be isolated from kimchi, and thus can be effectively used as a probiotic or food additive. Furthermore, the present invention relates to a composition for improving, preventing, or treating bone diseases or metabolic diseases, including Lactobacillus sakei CVL-001 strain (*Lactobacillus sakei*) or a culture medium thereof, wherein the Lactobacillus sakei strain isolated from kimchi and the culture medium thereof exhibit the effects of suppressing osteoclast differentiation, improving osteoporosis, suppressing adipocyte differentiation, and suppressing weight gain, and thus can be effectively used for the improvement, prevention, or treatment of osteoporosis or obesity related diseases.

## Description

### Technical Field

The present disclosure was made with the support of the Ministry of Science and ICT, Republic of Korea, under Project No. 2018R1A2B3004143, which was conducted by the Chonnam National R&BD Foundation in the research project named "Study on Nod2-mediated cell/tissue-specific defense system against pathogenic infection" under management of the National Research Foundation of Korea for the project named "Support project for mainstay researchers", from March 01, 2018 to 28 February, 2022.

This application claims priorities to and the benefits of Korean Patent Application Nos. 10-2019-0022670 and 10-2019-0022671 filed on 26 February, 2019 and Korean Patent Application No. 10-2020-0012763 filed on 03 February, 2020 in the Korean Intellectual Property Office, the contents of which are incorporated herein in their entireties by reference.

The present disclosure relates to a *Lactobacillus sakei* CVL-001 strain and an isolation method therefor and, more specifically, to a *Lactobacillus sakei* CVL-001 strain isolated and identified from kimchi.

In addition, the present disclosure relates to a composition comprising a *Lactobacillus sakei* CVL-001 strain or a culture liquid thereof for alleviating, preventing, or treating bone diseases or metabolic disease and, more specifically, to a composition comprising a *Lactobacillus sakei* CVL-001 strain deposited under accession number KCTC13816BP, or a culture liquid thereof for alleviating, preventing, or treating bone diseases or metabolic diseases.

### Background Art

Lactic acid bacteria are abundantly found in Korean traditional fermented foods such as kimchi. While existing in symbiosis with each other in the human digestive system, lactic acid bacteria are responsible for degrading fibers and composite proteins to afford important nutrients and maintaining the gut environment at acidic pH to inhibit the proliferation of harmful bacteria such as E. coli or *Chlostridium* sp. and to alleviate diarrhea and constipation, and play a role in vitamin synthesis and lowering blood cholesterol levels. Particularly, strongly binding to the mucosa and epithelial cells of the gut, lactic acid bacteria make great contribution to intestinal regulation.

Also, lactic acid bacteria are known to enhance the ability of macrophages to recognize and kill harmful gut bacteria by promoting the proliferation of macrophages and to exhibit an immunostimulatory effect by promoting the secretion of immune-related materials (Gabriela peridgon et al. J of food Protection 53: 404-411, 1990: Katsumasa sato et al., Microbiol Immunol., 32(7): 689-698, 1998) .

Lactobacillus spp. microorganisms, which perform homo- or heterolactic fermentation, are usually involved in fermenting dairy products and vegetables. Active research has recently been conducted into the development of Lactobacillus spp. microorganisms as probiotics, food additives, etc.

In recent years, the importance of the gut microbiota (hereinafter referred to as "GM") for both health and disease has been intensively studied. The GM includes trillions of bacteria which collectively contain 150-fold more genes than the human genome. GM is acquired at birth and, although being a distinct entity, has clearly coevolved with the human genome and can be considered a multicellular organ that communicates with and affects its host in numerous ways.

The composition of the GM is modulated by a number of environmental factors such as diet and antibiotic treatments. Molecules produced by the gut bacteria can be both beneficial and harmful and are known to affect endocrine cells in the gut, the enteric nervous system, gut permeability, and the immune system. A perturbed microbial composition has been postulated to be involved in a range of inflammatory conditions, within and outside the gut, including Crohn's disease, ulcerative colitis, rheumatoid arthritis, multiple sclerosis, diabetes, food allergies, eczema and asthma, as well as obesity and metabolic syndrome.

Climacteric women experience various menopausal syndromes. Particularly, a drop in estrogen in women at the time of menopause causes calcium resorption from bones to decrease bone mass. The increased bone loss makes the bone porous, leading to a high risk of osteoporosis. In many causes, it is not easy for women in a menopausal period to recognize post-menopausal symptoms because it takes a significant time for the symptoms to appear after onset of menopause. According to the Korean national health statistics of the Ministry of Health and Welfare, osteoporosis prevalence in persons at 30 years or more of age has been reported to be 1 % for men and 9 % for women, with 9-fold prevalence in women than men.

Bone fracture caused by osteoporosis accounts for a major health concern and results in a huge economic burden on health care systems. The lifetime risk of any osteoporotic fracture is high in the western world (around 50% for women and 20% for men) and fractures are associated with significant mortality and morbidity. Cortical bone accounts for approximately 80% of the bone in the body. Many studies have shown that cortical bone is the major determinant of bone strength and thereby fracture susceptibility. Bone loss after the age of 65 is mainly due to loss in cortical bone, but not trabecular bone (Lancet, 2010, May 15; 375(9727):1729-36) .

The skeleton is remodeled by bone forming osteoblasts (OBs) and bone resorbing osteoclasts (OCLs). Macrophage colony stimulating factor (MCSF) increases proliferation and survival of OCLs precursor cells as well as up-regulating expression of receptor activator of nuclear factor-κB (RANK) in OCLs. This allows RANK ligand (RANKL) to bind and start the signaling cascade that leads to OCL formation. The effect of RANKL can be inhibited by osteoprotegerin (OPG), which is a decoy receptor for RANKL.

Furthermore, metabolic diseases such as obesity, diabetes mellitus, and so on have tended to increase due to genetic and environmental factors, along with rapid economic growth and westernized dietary life. While studies for materials preventing and treating the diseases are ongoing, development of various functional foods has been required.

Obesity prevalence is increasing around the world while an interest in treating obesity has been rising due to metabolic complications caused by obesity. Anti-obesity drugs currently used are generally designed to suppress appetite by inducing satiety or to induce weight loss by lowering fat absorption. Satiety is induced by increasing a concentration of norepinephrine or serotonin at the synapses of the neurotransmitters or by stimulating serotonergic receptors or adrenergic receptors.

However, medicines developed for treatment of obesity exhibit serious adverse effects, compared to their pharmacological effects. There is therefore an urgent need for development of a substance that has a novel mechanism of action leading to an excellent anti-obesity effect with little side effects.

### Disclosure of Invention

### Technical Problem

Through intensive and thorough research, the present inventors succeeded in isolating and identifying a *Lactobacillus sakei* strain from kimchi samples collected from all parts of Korea and found that the *Lactobacillus sakei* strain isolated from kimchi and a culture liquid thereof have the effects of inhibiting osteoclast differentiation, alleviating osteoporosis diseases, inhibiting adipocyte differentiation, and suppressing weight gain.

A purpose of the present disclosure is therefore to provide a *Lactobacillus sakei* CVL-001 strain deposited under accession number KCTC13816BP.

Another purpose of the present disclosure is to provide a culture liquid of a *Lactobacillus sakei* CVL-001 strain deposited under accession number KCTC13816BP.

A further purpose of the present disclosure is to provide a method for isolation of a *Lactobacillus sakei* CVL-001 strain deposited under accession number KCTC13816BP, the method comprising a step of culturing a kimchi extract.

A still further purpose of the present disclosure is to provide a *Lactobacillus sakei* CVL-001 strain, deposited under accession number KCTC13816BP, which has the activity of alleviating, preventing, or treating a bone disease or a metabolic disease.

A still another purpose of the present disclosure is to provide a culture liquid of a *Lactobacillus sakei* CVL-001 strain, deposited under accession number KCTC13816BP, which has the activity of alleviating, preventing, or treating a bone disease or a metabolic disease.

A yet another purpose of the present disclosure is to provide pharmaceutical composition for prevention or treatment of a bone disease or a metabolic disease, the composition comprising a culture liquid of a *Lactobacillus sakei* CVL-001 strain deposited under accession number KCTC13816BP.

A yet further purpose of the present disclosure is to provide a food composition for alleviation of a bone disease or a metabolic disease, the composition comprising a culture liquid of a *Lactobacillus sakei* CVL-001 strain deposited under accession number KCTC13816BP.

An additional purpose of the present disclosure is to provide a use of a *Lactobacillus sakei* CVL-001 strain deposited under accession number KCTC13816BP in alleviating, preventing, and treating a bone disease or a metabolic disease.

### Solution to Problem

The present disclosure relates to a *Lactobacillus sakei* CVL-001 strain, an isolation method therefor, a composition comprising a *Lactobacillus sakei* CVL-001 strain or a culture liquid thereof for alleviating, preventing, or treating a bone disease or a metabolic disease.

The present inventors isolated strains from kimchi extracts, made a selection of Gram-positive, catalase-negative strains therefrom, and identified the selected strain as *Lactobacillus sakei* CVL-001. In addition, the strain or a culture liquid thereof was identified to inhibit osteoclast differentiation and have a therapeutic effect on a bone disease, with a suppression of weight gain and a decrease in blood glucose level observed in mice of high fat diet-induced obesity.

Below, a detailed description will be given of the present disclosure.

An aspect of the present disclosure pertains to a *Lactobacillus sakei* CVL-001 strain deposited under accession number KCTC13816BP.

The strain may include the 16S rRNA sequence represented by SEQ ID NO: 1.

Another aspect of the present disclosure pertains to a culture liquid of a *Lactobacillus sakei* CVL-001 strain deposited under accession number KCTC13816BP.

The strain may include the 16S rRNA sequence represented by SEQ ID NO: 1.

Another aspect of the present disclosure pertains to a method for isolating a *Lactobacillus sakei* CVL-001 strain deposited under accession number KCTC13816BP, the method comprising a step of culturing a kimchi extract.

The kimchi extract may be a kimchi-ground juice obtained by grinding kimchi and filtering the ground kimchi. For example, the kimchi extraction may be a diluted kimchi juice prepared by adding water to the kimchi-ground juice, but is not limited thereto.

The culturing step may include a first culturing step for incubating the kimchi extract; and a second culturing step for culturing a strain isolated in the first culturing step.

The first culturing step may be conducted in a medium containing 0.5 to 2% of calcium carbonate or 0.004 to 0.006% of bromocresol purple to discriminate lactic acid bacteria, for example, in a medium containing calcium carbonate, but with no limitations thereto.

The first culturing step may be conducted at 20 to 32°C for 36 to 60 hours, for example, at 30°C for 48 hours, but with no limitations thereto. Subsequently, colonies with a halo were picked and subjected to the second step.

The second culturing step may be a step in which lactic acid bacteria are selected from among the picked colonies and cultured. In detail, it may be a step in which selection is made of a Gram-positive, catalase-negative lactic acid bacterium from the isolated strains.

The second culturing step may be conducted at 20 to 32°C for 12 to 36 hours, for example, at for 30°C for 24 hours, but with no limitations thereto.

Another aspect of the present disclosure pertains to a *Lactobacillus sakei* CVL-001 strain, deposited under accession number KCTC13816BP, which has the activity of alleviating, preventing, or treating a bone disease. The strain may be isolated from kimchi, but with no limitations thereto.

A further aspect of the present disclosure pertains to a culture liquid of a *Lactobacillus sakei* CVL-001 strain, deposited under accession number KCTC13816BP, which has the activity of alleviating, preventing, or treating a bone disease.

A still further aspect of the present disclosure pertains to a pharmaceutical composition comprising a *Lactobacillus sakei* CVL-001 strain, deposited under accession number KCTC13816BP, or a culture liquid thereof for preventing or treating a bone disease.

The bone disease may be selected from osteoporosis, bone metastatic cancer, osteomalacia, rickets, fibrous osteitis, adynamic bone disease, metabolic disease, and periodontal disease, for example, osteoporosis, but with no limitations thereto.

The culture liquid may be a supernatant obtained by culturing the *Lactobacillus sakei* CVL-001 strain and removing the same, a concentrate thereof, a fraction thereof, or a lyophilisate thereof.

The culture liquid may be obtained as a result of culturing the *Lactobacillus sakei* CVL-001 strain for 12 to 30 hours, 12 to 24 hours, 18 to 30 hours, or 18 to 24 hours, for example, 22 to 26 hours, but with no limitations thereto. With a threshold given of 24 hours, the inhibitory activity against osteoclast differentiation is not increased in a culturing time-dependent manner when the culturing time exceeds the threshold.

For culturing the *Lactobacillus sakei* CVL-001 strain, Alpha-MEM medium may be utilized.

In detail, BME (Basal Media Eagle) medium, which is obtained as a result of a study on essential nutrients, inorganic salts, and vitamins necessary for cell growth, is a fundamental composition for MEM and DMEM, which are both widely used. MEM (Minimum Essential Media Eagle) medium is higher in amino acid concentration than BME and contains Earle's salts for buffering actions of CO₂/NaHCO₃. According to types of cells, a medium further supplemented with non-essential amino acids or a medium containing Hank's salts instead of Earle's salts may be used. Being supplemented with amino acids, vitamins, etc., Alpha MEM (MEM, Alpha modification) is useful for DNA transformation in animal cells.

The composition may contain the culture liquid at a concentration of 50 to 400, 50 to 350, 50 to 300, 50 to 250, 100 to 400, 100 to 350, 100 to 300, 100 to 250, 150 to 400, 150 to 350, 150 to 300, 150 to 250, 180 to 400, 180 to 350, or 180 to 300 µl/ml, for example, 180 to 250 µl/ml, but without limitations thereto.

The culture liquid may have a pH of 6.5 to 8.5, 6.5 to 8.0, 6.5 to 7.5, 7.0 to 8.5, or 7.0 to 8.0, for example, a pH of 7.0 to 7.5, but is not limited thereto.

As the lactic acid bacteria grow, metabolites such as lactic acid, etc. are produced to make the culture liquid acidic, which falls within a pH range unsuitable for cell culturing. On the basis of this situation, a pH of 7.4 is the most suitable for cell culturing.

The prevention and treatment of a bone disease could be achieved by inhibiting osteoclast differentiation, but with no limitations thereto.

A still another aspect of the present disclosure pertains to a pharmaceutical composition comprising a *Lactobacillus sakei* CVL-001 strain deposited under accession number KCTC13816BP or a culture liquid thereof for prevention or treatment of a metabolic disease.

The metabolic disease may be at least one selected from the group consisting of obesity, diabetes mellitus, hypertension, hyperlipidemia, cardiovascular disease, and hyperinsulinemia.

The pharmaceutical composition may comprise the *Lactobacillus sakei* CVL-001 strain at a concentration of 5 × 10⁵ to 5 × 10¹² CFU/ml, 5 × 10⁶ to 5 × 10¹¹ CFU/ml, or 5 × 10⁷ to 5 × 10¹⁰ CFU/ml, for example, 5 × 10⁸ to 5 × 10⁹ CFU/ml, but with no limitations thereto.

The culture liquid may be obtained by culturing the *Lactobacillus sakei* CVL-001 strain for 12 to 30 hours, 12 to 24 hours, 18 to 30 hours, or 18 to 24 hours, for example, 22 to 26 hours, but with no limitations thereto. With a threshold given of 24 hours, the effect of suppressing weight gain or reducing blood glucose levels is not significantly increased in a culturing time-dependent manner when the culturing time exceeds the threshold.

The culture liquid may be a culture supernatant obtained by culturing the *Lactobacillus sakei* CVL-001 strain and removing the same, a concentrate thereof, a fraction thereof, or a lyophilisate thereof.

The culture liquid may have a pH of 6.5 to 8.5, 6.5 to 8.0, 6.5 to 7.5, 7.0 to 8.5, or 7.0 to 8.0, for example, a pH of 7.0 to 7.5, but is not limited thereto.

As the lactic acid bacteria grow, metabolites such as lactic acid, etc. are produced to make the culture liquid acidic, which falls within a pH range unsuitable for cell culturing. On the basis of this situation, a pH of 7.4 is the most suitable for cell culturing.

The pharmaceutical composition of the present disclosure may be used as a pharmaceutical composition comprising a pharmaceutically effective amount of the *Lactobacillus sakei* CVL-001 strain culture liquid and/or a pharmaceutically acceptable carrier.

As used herein, the term "pharmaceutically effective amount" refers to an amount that allows the efficacy or activity of the *Lactobacillus sakei* CVL-001 strain culture liquid to be sufficiently achieved.

The pharmaceutically acceptable carrier contained in the pharmaceutical composition of the present disclosure may be any one that is usually used in formulations. Examples of the carrier include lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxy benzoate, propyl hydroxy benzoate, talc, magnesium stearate, mineral oil, and the like, but are not limited thereto. In addition to the carriers, the pharmaceutical composition of the present disclosure may further contain a lubricant, a humectant, a sweetener, a flavorant, an emulsifier, a suspending agent, a preservative, and so on.

The pharmaceutical composition according to the present disclosure may be administered to mammals including humans via various routes. All possible routes of administration may be contemplated, including, for example, oral, dermal, intravenous, intramuscular, subcutaneous, and other routes, with preference for an oral route.

A suitable dose of the pharmaceutical composition of the present disclosure may vary depending on pharmaceutical formulation methods, administration methods, the patient's age, body weight, sex, severity of diseases, diet, administration time, administration route, an excretion rate and sensitivity. Physicians with average skill may easily determine and diagnose dosage level of medicine effective for desirably treating or preventing target disorders or diseases.

The pharmaceutical composition of the present disclosure may be formulated into a unit dose form and a multi-dose form according to methods that can be implemented by a person skilled in the art, using a pharmaceutically acceptable carrier and/or vehicle. In this regard, the formulation may be in the form of a solution in an oil or aqueous medium, a suspension or an emulsion, an extract, a pulvis, a granule, a tablet, a capsule, or a gel (e.g., hydrogel) and may further comprise a dispersant or a stabilizer.

A yet further aspect of the present disclosure pertains to a food composition comprising a *Lactobacillus sakei* CVL-001 strain deposited under accession number KCTC13816BP or a culture liquid thereof for alleviation of a bone disease.

The bone disease may be selected from osteoporosis, bone metastatic cancer, osteomalacia, rickets, fibrous osteitis, adynamic bone disease, metabolic disease, and periodontal disease, for example, osteoporosis, but with no limitations thereto.

The culture liquid may be a supernatant obtained by culturing the *Lactobacillus sakei* CVL-001 strain and removing the same, a concentrate thereof, a fraction thereof, or a lyophilisate thereof.

The culture liquid may be obtained as a result of culturing the *Lactobacillus sakei* CVL-001 strain for 12 to 36 hours, 12 to 30 hours, 12 to 24 hours, 18 to 36 hours, 18 to 30 hours, or 18 to 24 hours, for example, 22 to 26 hours, but with no limitations thereto.

The composition may contain the culture liquid at a concentration of 20 to 400, 20 to 350, 20 to 300, 20 to 250, 50 to 400, 50 to 350, 50 to 300, 50 to 250, 100 to 400, 100 to 350, 100 to 300, 100 to 250, 150 to 400, 150 to 350, 150 to 300, 150 to 250, 180 to 400, 180 to 350, or 180 to 300 µl/ml, for example, 180 to 250 µl/ml, but without limitations thereto.

The culture liquid may have a pH of 6.5 to 8.5, 6.5 to 8.0, 6.5 to 7.5, 7.0 to 8.5, or 7.0 to 8.0, for example, a pH of 7.0 to 7.5, but is not limited thereto.

The alleviation of a bone disease could be achieved by inhibiting osteoclast differentiation, but with no limitations thereto.

A yet another aspect of the present disclosure pertains to a health functional food composition comprising a *Lactobacillus sakei* CVL-001 strain deposited under accession number KCTC13816BP or a culture liquid thereof for alleviation of a metabolic disease.

The metabolic disease may be at least one selected from the group consisting of obesity, diabetes mellitus, hypertension, hyperlipidemia, cardiovascular disease, and hyperinsulinemia.

The health functional food composition may comprise the *Lactobacillus sakei* CVL-001 strain at a concentration of 5 × 10⁵ to 5 × 10¹² CFU/ml, 5 × 10⁶ to 5 × 10¹¹ CFU/ml, or 5 × 10⁷ to 5 × 10¹⁰ CFU/ml, for example, 5 × 10⁸ to 5 × 10⁹ CFU/ml, but with no limitations thereto.

The culture liquid may be obtained by culturing the *Lactobacillus sakei* CVL-001 strain for 12 to 36 hours, 12 to 30 hours, 12 to 24 hours, 18 to 36 hours, 18 to 30 hours, or 18 to 24 hours, for example, 22 to 26 hours, but with no limitations thereto.

The culture liquid may be a culture supernatant obtained by culturing the *Lactobacillus sakei* CVL-001 strain and removing the same, a concentrate thereof, a fraction thereof, or a lyophilisate thereof.

The culture liquid may have a pH of 6.5 to 8.5, 6.5 to 8.0, 6.5 to 7.5, 7.0 to 8.5, or 7.0 to 8.0, for example, a pH of 7.0 to 7.5, but is not limited thereto.

When used as a food additive, the food composition of the present disclosure invention may be added as it is or may be used in combination with another food or food ingredient, and can be suitably applied in conventional manners. In preparing foods or beverages, the food composition may be generally added in an amount of 15 % by weight or less and preferably in an amount of 10 % by weight, based on the weight of the raw materials.

No particular limitations are imparted to types of foods to which the food composition can be applied. Examples of the foods added include meats, sausages, breads, chocolate, candies, snacks, confectionery, pizza, ramen, other types of noodles, gums, dairy products including ice creams, various types of soups, beverages, teas, drinks, alcohol drinks and vitamin complexes, and in a common sense, all types of foods. The beverages may contain various flavorants or natural carbohydrates as additional ingredients. Examples of the natural carbohydrates include monosaccharides, such as glucose and fructose, disaccharides, such as maltose and sucrose, natural sweeteners, such as dextrin and cyclodextrin, and synthetic sweeteners, such as saccharin and aspartame. The amount of the natural carbohydrates can be appropriately determined by a person skilled in the art.

In addition, the composition of the present disclosure may contain various nutrients, vitamins, electrolytes, flavorants, colorants, pectic acid or salts thereof, alginic acid or salts thereof, organic acids, protective colloidal thickeners, pH adjusters, stabilizers, preservatives, glycerin, alcohol, carbonating agents used in carbonated drinks, etc. Moreover, the food composition of the present disclosure may contain fruit flesh for the preparation of natural fruit juices, fruit juice beverages and vegetable juices. These components may be used alone or in combination. The contents of these additives in the composition can be appropriately selected by a person skilled in the art.

### Advantageous Effects of Invention

The present disclosure relates to a *Lactobacillus sakei* CVL-001 strain and an isolation method therefor. The strain can be isolated from kimchi and, as such, can be advantageously used in a probiotic agent or a food additive.

In addition, the present disclosure relates to a composition comprising a *Lactobacillus sakei* CVL-001 strain or a culture liquid thereof for alleviating, preventing, or treating a bone disease. Exhibiting the effect of inhibiting osteoclast differentiation, alleviating osteoporotic diseases, and suppressing adipogenic differentiation and weight gain, the *Lactobacillus sakei* strain isolated kimchi and a culture liquid thereof can find advantageous applications in alleviating, preventing, or treating osteoporosis or obesity disease.

### Brief Description of Drawings

FIG. 1a is a photographic image showing an inhibitory effect of a *Lactobacillus sakei* CVL-001 strain culture liquid on osteoclast differentiation.
FIG. 1b is a graph showing an inhibitory effect of a *Lactobacillus sakei* CVL-001 strain culture liquid on osteoclast differentiation.
FIG. 2 is a photographic image showing western blots that account for an inhibitory effect of a *Lactobacillus sakei* CVL-001 strain culture liquid on the phosphorylation of osteoclast differentiation-inducing proteins.
FIG. 3 shows graphs that account for inhibitory effects of a *Lactobacillus sakei* CVL-001 strain culture liquid on the expression of osteoclast differentiation-inducing genes.
FIG. 4 is a graph showing the increasing effect of a *Lactobacillus sakei* CVL-001 strain and a culture liquid thereof on bone mineral density in osteoporosis-induced mouse models.
FIG. 5 is a graph showing an inhibitory effect of a *Lactobacillus sakei* CVL-001 strain culture liquid on adipocyte differentiation.
FIG. 6 is a plot showing inhibitory effects of a *Lactobacillus sakei* CVL-001 strain on weight gain in high fat diet-fed experimental groups with time.
FIG. 7 is a graph showing reductive effects of a *Lactobacillus sakei* CVL-001 strain on epididymal fat weight in high fat diet-fed experimental groups.
FIG. 8 is a plot showing inhibitory effects of a *Lactobacillus sakei* CVL-001 strain culture liquid on weight gain in high fat diet-fed experimental groups with time.
FIG. 9 is a graph showing reductive effects of a *Lactobacillus sakei* CVL-001 strain culture liquid on epididymal fat weight in high fat diet-fed experimental groups.
FIG. 10 is a plot showing antidiabetic effects of a *Lactobacillus sakei* CVL-001 strain culture liquid in high fat diet-fed experimental groups as measured by a glucose tolerance test.
FIG. 11 is a plot showing antidiabetic effects of a *Lactobacillus sakei* CVL-001 strain in high fat diet-fed experimental groups as measured by a glucose tolerance test.
FIG. 12 is a plot showing antidiabetic effects of a *Lactobacillus sakei* CVL-001 strain culture liquid in high fat diet-fed experimental groups as measured by an insulin tolerance test.
FIG. 13 is a plot showing antidiabetic effects of a *Lactobacillus sakei* CVL-001 strain in high fat diet-fed experimental groups as measured by an insulin tolerance test.

### Best Mode for Carrying out the Invention

The present disclosure is concerned with a *Lactobacillus sakei* CVL-001, deposited under accession number KCTC13816BP, having activity of alleviating, preventing, or treating a bone disease.

### Mode for Carrying out the Invention

A better understanding of the present disclosure may be obtained via the following examples which are set forth to illustrate, but are not to be construed as limiting the present disclosure.

Unless stated otherwise, "%", used to indicate concentrations of particular substances, stands for (wt./wt.) % for solid/solid, (wt./vol.) % for solid/liquid, and (vol./vol.) % for liquid/liquid throughout the specification.

### EXAMPLE 1: Isolation of Strain

Napa cabbage kimchi just after being prepared was collected from all parts of Korea. While being stored at 6±0.5°C from week 0 to week 5, the collected kimchi was used as kimchi samples for isolating lactic acid bacteria. A hand blender (Hanil Co, Korea) was used to grind 500 g of the kimchi, followed by filtration through sterile gauze. The filtrate was diluted with sterile water in a serial manner from 10¹ to 10⁷ fold. Finally, the 10⁷-fold diluted kimchi juice was spread over an agar.

The diluted kimchi juice was spread over an MRS agar (Difco Co., France) containing 2% of calcium carbonate (CaCO₃) and incubated at 30°C for 48 hours before picking up colonies with halo. The picked colonies were subjected to Gram staining (Gram stain kit, BD Co., USA) and catalase testing (Biomerieux Co., France) to tentatively select Gram-positive, catalase-negative colony as lactic acid bacteria.

The lactic acid bacterium strains thus isolated were inoculated into an MRS broth, incubated at 30°C for 24 hours, and added with 25% (v/v) of glycerol to prepare glycerol stocks which were stored at -70°C until use.

### EXAMPLE 2: Identification of Lactic Acid Bacteria

The finally selected lactic acid bacterium *Lactobacillus sakei* CVL-001 was identified by 16S rRNA sequencing. After purely isolated, the strains spread on MRS plates were delivered via refrigerated parcel service to SolGent (Daejeon, Korea) in which the strains were analyzed by 16S rRNA sequencing with a universal primer set of 27F (5'-AGAGTTTGATCCTGGCTCAG-3') and 1492R (5'-GGTTACCTTGTTACGACTT-3') .

The isolated strain *Lactobacillus sakei* CVL-001 was determined to have the 16S rRNA coding nucleotide sequence consisting of a total of 1,439 bp shown in Table 1, below.

**[TABLE 1]**

| SEQ ID NO: | Name | Sequence |
|---|---|---|
| 1 | 16S rRNA coding nucleotide sequence of *Lactobacill us sakei* CVL-001 | GCAGTCGAACGCACTCTCGTTTAGATTGAAGGAGCTTGCTCCTGATTGATAAA CATTTGAGTGAGTGGCGGACGGGTGAGTAACACGTGGGTAACCTGCCCTAAAG TGGGGGATAACATTTGGAAACAGATGCTAATACCGCATAAAACCTAACACCGC ATGGTGTAGGGTTGAAAGATGGTTTCGGCTATCACTTTAGGATGGACCCGCGG TGCATTAGTTAGTTGGTGAGGTAAAGGCTCACCAAGACCGTGATGCATAGCCG ACCTGAGAGGGTAATCGGCCACACTGGGACTGAGACACGGCCCAGACTCCTAC GGGAGGCAGCAGTAGGGAATCTTCCACAATGGACGAAAGTCTGATGGAGCAAC GCCGCGTGAGTGAAGAAGGTTTTCGGATCGTAAAACTCTGTTGTTGGAGAAGA ATGTATCTGATAGTAACTGATCAGGTAGTGACGGTATCCAACCAGAAAGCCAC GGCTAACTACGTGCCAGCAGCCGCGGTAATACGTAGGTGGCAAGCGTTGTCCG GATTTATTGGGCGTAAAGCGAGCGCAGGCGGTTTCTTAAGTCTGATGTGAAAG CCTTCGGCTCAACCGAAGAAGTGCATCGGAAACTGGGAAACTTGAGTGCAGAA GAGGACAGTGGAACTCCATGTGTAGCGGTGAAATGCGTAGATATATGGAAGAA CACCAGTGGCGAAGGCGGCTGTCTGGTCTGTAACTGACGCTGAGGCTCGAAAG CATGGGTAGCAAACAGGATTAGATACCCTGGTAGTCCATGCCGTAAACGATGA GTGCTAGGTGTTGGAGGGTTTCCGCCCTTCAGTGCCGCAGCTAACGCATTAAG CACTCCGCCTGGGGAGTACGACCGCAAGGTTGAAACTCAAAGGAATTGACGGG GGCCCGCACAAGCGGTGGAGCATGTGGTTTAATTCGAAGCAACGCGAAGAACC TTACCAGGTCTTGACATCCTTTGACCACTCTAGAGATAGAGCTTTCCCTTCGG GGACAAAGTGACAGGTGGTGCATGGTTGTCGTCAGCTCGTGTCGTGAGATGTT |
| | | GGGTTAAGTCCCGCAACGAGCGCAACCCTTATTACTAGTTGCCAGCATTTAGT TGGGCACTCTAGTGAGACTGCCGGTGACAAACCGGAGGAAGGTGGGGACGACG TCAAATCATCATGCCCCTTATGACCTGGGCTACACACGTGCTACAATGGATGG TACAACGAGTTGCGAGACCGCGAGGTTTAGCTAATCTCTTAAAACCATTCTCA GTTCGGATTGTAGGCTGCAACTCGCCTACATGAAGCCGGAATCGCTAGTAATC GCGGATCAGCATGCCGCGGTGAATACGTTCCCGGGCCTTGTACACACCGCCCG TCACACCATGAGAGTTTGTAACACCCAAAGCCGGTGAGGTAACCCTTCGGGGA GCCAGCCG |
| 2 | 27F forward primer | AGAGTTTGATCCTGGCTCAG |
| 3 | 1492R backward primer | GGTTACCTTGTTACGACTT |

With respect to the nucleotide sequence obtained above, sequence similarity search was run with the Blast program (www.ncbi.nlm.nhi.gov) for the registered database (GenBank database). As a result, the strain was finally identified as *Lactobacillus sakei* LZ217 because of a similarity of 99% therewith.

In light of the results obtained above, that is, morphological traits, sugar metabolism ability, and 16S rRNA sequencing results, the *Lactobacillus sakei* CVL-001 strain with excellent mannitol productivity and viability (predominancy) in kimchi juice was identified as *Lactobacillus sakei* LZ217.

The isolated strain was termed *Lactobacillus sakei* CVL-001 and deposited with the Korean Collection for Type Culture located at 823, Shinjung-dong, Jeongeup City, Jeollabuk-do, Korea.

### EXAMPLE 3: Preparation of Lactobacillus sakei CVL-001 Strain Culture Liquid

A culture for use in application to cells was obtained by culturing *Lactobacillus sakei* CVL-001 for 24 hours in alpha-MEM medium, centrifuging the cell culture, and adjusting the separated supernatant into a pH of 7.4.

### EXAMPLE 4: Assay for Inhibitory Activity of Lactobacillus sakei CVL-001 Strain Culture Liquid against Osteoclast Differentiation

Mouse macrophages were seeded at a density of 2×10⁵ cells/well into 12-well plates and incubated for 24 hours. After the medium was changed with a medium supplemented with fetal bovine serum (hereinafter referred to as "FBS"), 1% penicillin-streptomycin (hereinafter referred to as "PS"), and macrophage colony stimulating factor (hereinafter referred to as "M-CSF"), the cells were incubated with the culture liquid (50, 100, and 200 µl/ml) for 2 hours. Then, the cells were treated with 100 ng/ml RANKL (receptor activator of nuclear factor kappa-B ligand) for 24 hours and allowed to differentiate for 6 days in the manner described above.

Subsequently, a chromogenic substrate for the cytochemical enzyme marker tartrate-resistant acid phosphatase (hereinafter referred to as "TRAP") was added to stain the nuclei. Multinucleated cells with three or more nuclei were observed and imaged.

When applied to macrophages, RANKL binds to RANK to cause differentiation into TRAP positive cells. Stimulation of TRAP-positive cells with an inflammatory factor such as RANKL, TNF-α, etc. renders cell fusion into multinuclear TRAP positive cells.

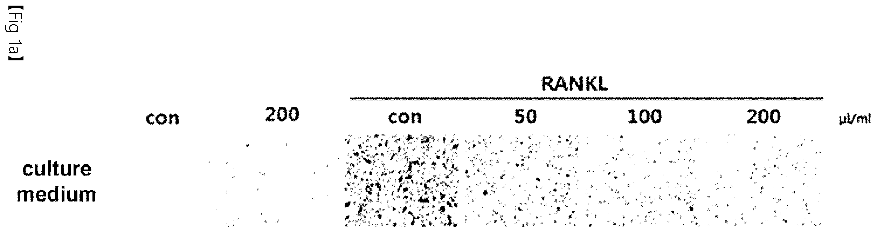

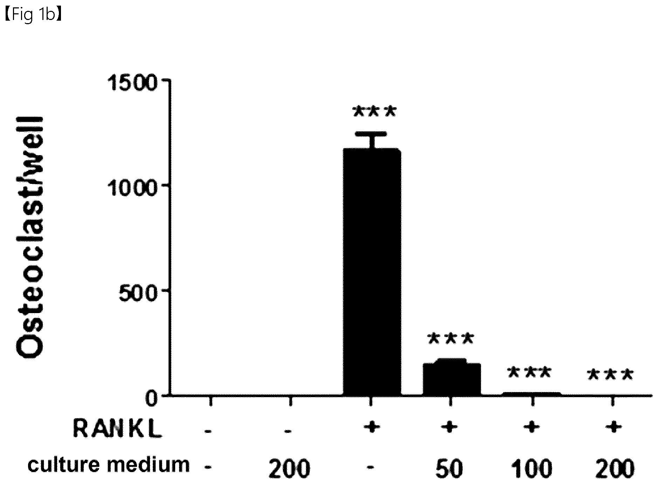

As shown in FIGS. 1a and 1b and Table 2, treatment of macrophage with RANKL increased differentiation into osteoclasts and the culture liquid was observed to significantly decrease the count of osteoclasts in a dose-dependent manner. From the result, it was understood that the *Lactobacillus sakei* CVL-001 culture liquid can inhibit osteoclast differentiation.

**[TABLE 2]**

| RANKL | - | - | + | + | + | + |
|---|---|---|---|---|---|---|
| Culture liquid | - | 200 | - | 50 | 100 | 200 |
| Counts of osteoclast (Avg.) | 0 | 0 | 1,165 | 151 | 10 | 0 |

### EXAMPLE 5: Assay for Inhibitory Activity of Lactobacillus sakei CVL-001 Strain Culture Liquid against Expression of Osteoclast Differentiation-Inducing Protein

Examination was made of the MAPK (mitogen-activated protein kinase) and NF-κB signaling mechanism when mouse macrophages were treated with RANKL and the culture liquid. To this end, Mouse macrophages were seeded at a density of 4×10⁵ cells/well into 12-well plates and incubated for 24 hours. After the medium was changed with a medium supplemented with FBS, 1% PS, and M-CSF, the *Lactobacillus sakei* CVL-001 strain culture liquid (100 µl/ml) was incubated for 2 hours. Then, the cells were treated with 100 ng/ml RANKL for 0, 5, 15, and 30 min.

After the medium was removed, proteins were extracted with a protein lysis buffer and quantitated and 30 µg of the proteins was separated in SDS-PAGE gel. The proteins were transferred onto a membrane, incubated with primary and secondary antibodies (p-jnk (Cell Signaling Technology, #9251S), p-p38 (Cell Signaling Technology, 9211S), p-ERK (Santa Cruz, sc-7383), Ikbalpha (Cell Signaling Technology, 9242S), p-p65 (Cell Signaling Technology, 3031S)), sequentially. Color development was made using a kit (BIO-RAD, ECL solution detection kit) and measured by a device (Chemidoc).

RANKL binds to RANK to upregulate the expression of MAPK and NF-κB via TRAF6 and induce the activation of various transcription factors, leading to the promotion of osteoclast differentiation. Examination was thus made to see whether the *Lactobacillus sakei* culture liquid intervenes with the mechanisms to inhibit osteoclast differentiation. In the regard, phosphorylation of the MAPK proteins ERK, JNK, and p38 was analyzed by western blotting.

As a result, increased phosphorylation levels of JNK, P38, and ERK were observed 5 and 15 min after incubation with RANKL whereas the MAPK proteins were dephosphorylated by treatment with the culture liquid. In addition, inhibition was observed on both the degradation of IkB alpha and the phosphorylation of p65.

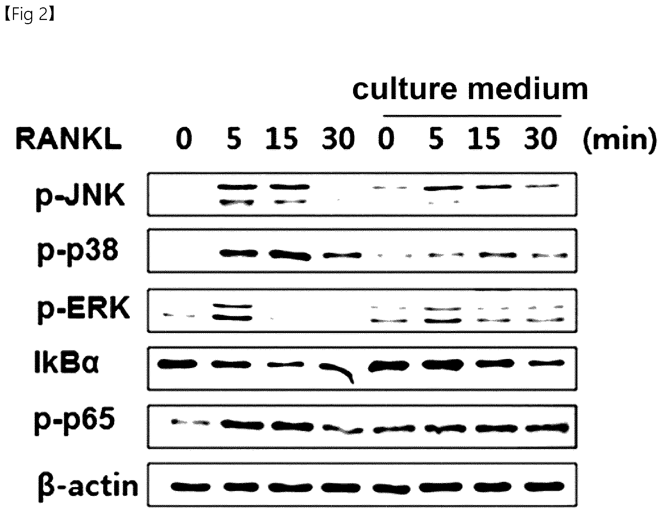

As is understood from the data of FIG. 2, the *Lactobacillus sakei* culture liquid inhibits the phosphorylation of JNK, P38, and ERK, thereby suppressing differentiation into osteoclasts.

### EXAMPLE 6: Assay for Effect of Lactobacillus sakei Culture Liquid on Expression of Osteoclast Differentiation-Inducing Gene

After being treated with the *Lactobacillus sakei* culture liquid, the mouse macrophages were assayed for gene expression of TRAP, DC-STAMP, Cathepsin K, and NFATc1, which are genes involved in osteoclastogenesis, through real-time PCR. Bone marrow-derived macrophages (hereinafter referred to as "BMDMs") were seeded at a density of 1×10⁵ cells/well into 12-well plates and incubated for 24 hours. Next, the cells were treated with a FBS-free medium supplemented with 1% PS only for 1 hour and then with 100 µl/ml *Lactobacillus sakei* culture liquid for 2 hours, followed by incubation with RANKL for 24 hours. In this manner, the cells were subjected to differentiation for 3 days before RNA isolation from the cells with the aid of TRIzol solution. On the basis of RNA quantitation, RT premix was used to synthesize cDNA which was then amplified by real-time PCR using primers.

The primers used were designed for Mouse TRAP, DC-STAMP, Cathepsin K, and NFATc1, which were assayed in relative amounts to the control gene GAPDH.

Macrophages undergo differentiation into TRAP-positive, multinucleated osteoclasts when treated When treated with RANKL, which binds to RANK. In addition, treatment with RANKL activates NFATc1, which is a transcription factor playing an important role in differentiation into osteoclasts, and upregulates the expression of TRAP, Cathepsine K, and DC-STAMP, which are involved in osteoclastogenesis.

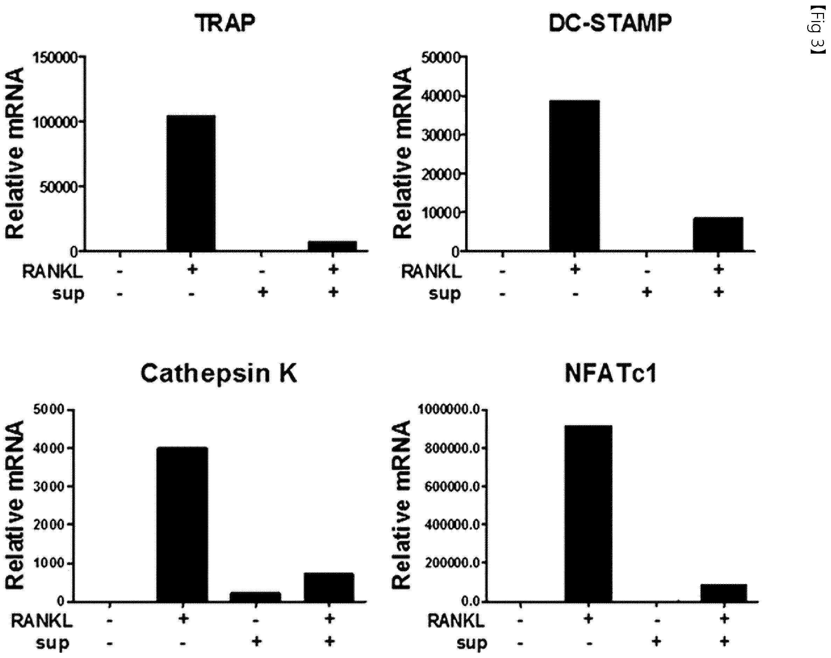

As can be seen in FIG. 3 and Table 3, the expression levels of TRAP, DC-STAMP, Cathepsin K, and NFATc1, which were elevated by treatment with RANKL, were decreased by treatment with *Lactobacillus sakei* culture liquid. These data imply that the *Lactobacillus sakei* culture liquid inhibits the activity of the transcription factor NFATc1 to downregulate the expression of TRAP, DC-STAMP, and Cathepsin K, which are genes involved in osteoclastogenesis, thereby suppressing osteoclast differentiation.

**[TABLE 3]**

| RANKL | - | + | - | + |
|---|---|---|---|---|
| Culture liquid | - | - | + | + |
| TRAP | 1 | 104,133 | 201.9 | 7,122 |
| DC-STAMP | 1 | 38,698 | 72 | 8,539 |
| Cathepsin K | 1 | 3,983 | 210 | 709 |
| NFATc1 | 1 | 913,838 | 922 | 85,284 |

### EXAMPLE 7: Construction of Osteoporosis Animal Model by Mouse Ovariectomy

C57BL/6 female mice (7 weeks old) were housed in plastic cages maintained at a temperature of 22±2°C and a relative humidity of 50±10% with a photoperiod of 12-h light and 12-h dark. The mice were acclimated to the same environment for about one week before ovariectomy.

The mice were anesthetized using an intramuscular injection of Zoletil and Lumpun, after which the ovariectomy region was shaved and sterilized. A 1-cm incision was made on the skin. Then, the ovary was detected along the uterus with care to exert no injuries on other organs and ligated with a suture so as to resect the opposite ovaries. After ovariectomy, the organs were each repositioned within the abdomen and the incision was sutured with a thread. From day 10, a therapeutic substance was administered for 8 weeks.

Administration of lactic acid bacteria: After preculture and main culture, *Lactobacillus sakei* was divided into aliquots of 1×10⁷, 1×10⁸, and 1×10⁹ cells/ml and orally administered.

Administration of culture liquid: After preculture and main culture, *Lactobacillus sakei* was divided into aliquots of 1×10⁷, 1×10⁸, and 1×10⁹ cells/ml. The cell aliquots were 10-fold diluted and cultured in an MRS medium at 30°C for 24 hours. The cells were settled by centrifugation, and the culture liquid supernatant was collected and adjusted into a pH of 7.4 before oral administration at a dose of 200 µl.

Test groups were divided as follows:
C57BL/6 mice; 80 heads
① G1: non-ovariectomized sham, 10 heads, MRS medium (control) administered
② G2: non-ovariectomized sham, 10 heads, culture liquid administered
③ G3: non-ovariectomized sham, 10 heads, Lactobacillus sakei administered at a dose of 1×10⁹ cells/ml
④ G4: ovariectomized test group (OVX), 10 heads, MRS medium (control) administered
⑤ G5: ovariectomized test group (OVX), 10 heads, culture liquid (1/4 diluted) administered
⑥ G6: ovariectomized test group (OVX), 10 heads, culture liquid (1/2 diluted) administered
⑦ G7: ovariectomized test group (OVX), 10 heads, culture liquid (stock) administered
⑧ G8: ovariectomized test group (OVX), 10 heads, Lactobacillus sakei administered at a dose of 1×10⁷ cells/ml
⑨ G9: ovariectomized test group (OVX), 10 heads, Lactobacillus sakei administered at a dose of 1×10⁸ cells/ml
⑩ G10: ovariectomized test group (OVX), 10 heads, Lactobacillus sakei administered at a dose of 1×10⁹ cells/ml

### EXAMPLE 8: Evaluation of Bone Mineral Density in Osteoporosis-Induced Mice According to Administration of Lactobacillus sakei and Culture Liquid thereof

Bone mineral densities were measured using microcomputed tomography (micro-CT), which can provide high-definition images than simple radiography or computed tomography.

After completion of the experiment, the mouse legs were fixed with formalin, and analyzed for bone mineral density in the Laboratory Animal Center of Daegu-Gyeongbuk Medical Innovation Foundation.

Ovariectomized mice are known to decrease in bone mineral density due to estrogen inefficiency and as such, are widely used for studying osteoporosis diseases.

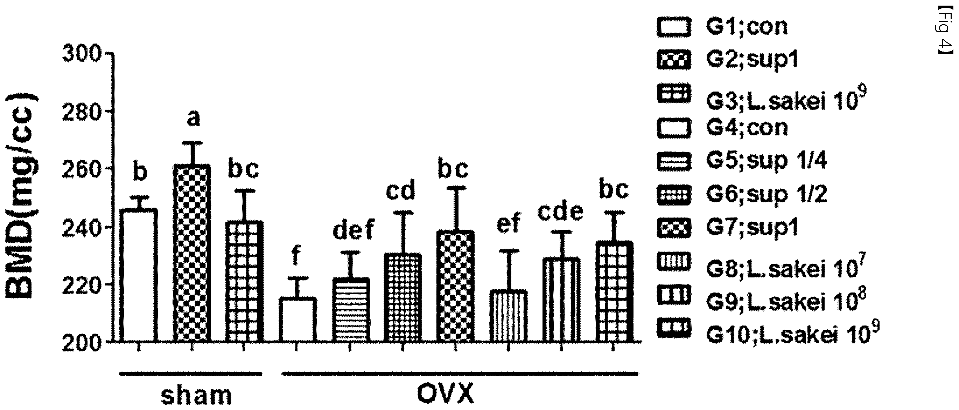

As can be seen in FIG. 4 and Table 4, the ovariectomized mouse group (G4) was observed to significantly decrease in bone mineral density, compared to the sham groups. Meanwhile, the *Lactobacillus sakei* strain and the culture liquid thereof increased the bone mineral density in a dose-dependent manner. Particularly, the groups to which the culture liquid stock (G7) and a high concentration of the bacteria (G10) were administered recovered the bone mineral density to levels as high as those in the non-ovariectomized sham groups.

**[TABLE 4]**

| | G1 | G2 | G3 | G4 | G5 | G6 | G7 | G8 | G9 | G10 |
|---|---|---|---|---|---|---|---|---|---|---|
| Bone Mineral Density (mean) (mg/cc) | 245.9 | 260.9 | 241.7 | 215.3 | 221.7 | 230.3 | 238.6 | 217.6 | 229.1 | 234. 8 |

### EXAMPLE 9: Assay for Inhibitory Effect of Culture Liquid of Lactobacillus sakei CVL-001 Isolated from Kimchi on Adipocyte Differentiation (in vitro)

### 9-1. Preparation of Lactobacillus sakei CVL-001 culture liquid

*Lactobacillus sakei* CVL-001 was cultured at a density of 1×10⁸ CFU/ml in DMEM medium for 24 hours, followed by centrifugation. The supernatant thus obtained was adjusted into a pH of 7.4 to afford a *Lactobacillus sakei* CVL-001 culture liquid.

### 9-2. Adipose differentiation using undifferentiated preadipocyte strain 3T3-L1

Preadipocyte 3T3-L1 purchased from ATCC (American type culture collection, USA) was maintained by passage in DMEM supplemented with 10% bovine calf serum (BCS) and 1% PS. The day at which the cells reached 100% confluence after being seeded into plates was designated day -2. While the cells were cultured for an additional two days, the cell cycle was arrested at G1 phase in all the cells cultured in the plates.

For day 0, the cells were allowed to start differentiation in the presence of IBMX (3-isobutyl-1-methylxanthine), dexamethasone, and insulin (MDI). On day 2, the medium was changed with a medium containing insulin only. From day 4, the medium was changed with 10% fetal bovine serum (FBS)-supplemented medium every two days until differentiation.

### 9-3. Assay for Inhibitory Activity of Lactobacillus sakei CVL-001 Culture Liquid against Adipocyte Differentiation (Oil Red O Staining)

Adipocyte differentiation was induced when bovine calf serum (BCS) in the medium composition was substituted with FBS. 3T3-L1 cells were seeded at a density of 5×10⁵ cells/well into 12-well plates containing a medium supplemented with 10% FBS and 1% PS. On two days after the cells reached 100% confluence, that is, on day 0, the cells were treated with the culture liquid (12.5, 25.0, and 50.0 %) for 2 hours and then with the adipogenesis inducer MDI.

The culture liquid was prepared as a result of culturing *L. sakei* at a concentration of 1 × 10⁸ cfu/ml in DMEM (High glucose) + FBS (10%) + PS (1%) medium for 24 hours, and dilution of the culture liquid was conducted with DMEM medium.

On day 2, the cells were incubated with the culture liquid (12.5, 25.0, and 50.0 %) for 2 hours while the medium was changed, followed by treatment with insulin. On day 4, the cells were incubated with the culture liquid (12.5, 25.0, and 50.0 %) while the medium was changed. The same procedure was repeated every two days until differentiation.

When differentiated adipocytes were observed, the medium was changed with 4% formalin to fix the cells for 10 min. For adipocyte staining, the cells were washed twice with D.W. and incubated with a mixture of 6 : 4 Oil red O staining : D.W. for 30 min. Washing with D.W. was followed by microscopic observation. Then, Oil red O was extracted with 100% isopropanol and quantitative analysis was performed by reading absorbance at 510 nm.

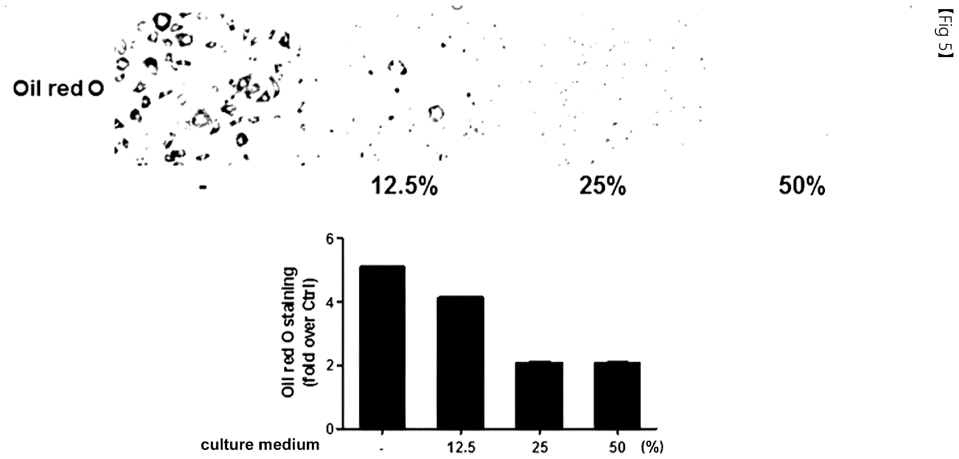

As is understood from the data of FIG. 5 and Table 5, 3T3-L1 cells underwent adipogenic differentiation when MDI was added to the cell culture medium. In this study result, exposure of 3T3-L1 cells to MDI increased adipogenic differentiation whereas the culture liquid was observed to significantly reduce counts of adipocytes in a dose-dependent manner.

**[TABLE 5]**

| Culture liquid (%) | - | 12.5 | 25.0 | 50.0 |
|---|---|---|---|---|
| Degree of adipogenic differentiation | 5.7052 | 4.1410 | 2.0922 | 2.0940 |

Taken together, the data imply that the *Lactobacillus sakei* CVL-001 culture liquid can inhibit adipogenic differentiation.

### EXAMPLE 10: Construction of Obesity Mouse Animal Model for Assaying Reductive Effect on Obesity (in vivo)

### 10-1. Preparation of live Lactobacillus sakei CVL-001

Live bacteria to be applied to mice were prepared. In brief, the bacteria were cultured for 24 hours at 30°C in MRS agar plates, and a single colony thus formed was picked and pre-cultured in MRS broth. The pre-culturing was conducted in 5 ml of the medium at 30°C for 24 hours while shaking at 150 rpm. Subsequently, a 10-fold dilution of the culture was amplified for 3 hours in the same conditions.

In order to keep a balance between cell counts, the cultures finally obtained were diluted in PBS until the optical density at 600 nm (OD600) reached 0.6 as measured by a spectrophotometer. The O.D. value accounts for the existence of the lactic acid bacteria at a concentration of 0.89×10⁹ CFU/ml. With the administration of 200 µl to each mouse (1×10⁸ CFU/mouse, 1×10⁹ CFU/mouse) in mind, live bacterial media were prepared by centrifugation at 3,000 RPM for 15 min.

### 10-2. Preparation of Lactobacillus sakei CVL-001 culture liquid

An MRS medium in which the amplification of *Lactobacillus sakei* had been completed was 10-fold diluted was incubated at 30°C for 24 hours while shaking at 150 rpm. After centrifugation at 3,000 RPM for 15 min, the supernatant was separated and adjusted into a pH of 7.4. Filtration through 0.45 µm membrane filter paper was conducted before storage at 4°C.

### 10-3. Assay for anti-obesity activity of Lactobacillus sakei CVL-001 strain and culture liquid (in vivo)

To C57BL/6 male mice at 7 weeks of age were administered the live bacteria (single daily dose 200 µl/subject), the culture liquid (single daily dose 200 µl/subject), and a high fat diet. Subsequently, the mice were weighed every two weeks. At week 11, the mice were measured for blood sugar levels. At week 14, an autopsy was performed on the mice, after which blood was sampled and the organs were weighed.

Information on the groups used for the strain test was as follows:
G1: Control group (normal diet fed), PBS (phosphate buffered saline) orally administered (n=6)
G2: Control group, 10⁹ *L. sakei* orally administered (n=6)
G3: High fat group (high fat diet fed), PBS orally administered (n=8)
G4: High fat group, 10⁸ *L. sakei* orally administered (n=8)
G5: High fat group, 10⁹ *L. sakei* orally administered (n=8)

Information on groups used for culture liquid testing was as follows:
G1: Control group (normal diet fed), MRS orally administered (n=6)
G2: Control group, culture liquid (stock) orally administered (n=6)
G3: High fat group (high fat diet fed), MRS orally administered (n=8)
G4: High fat group, culture liquid (1/2 diluted) orally administered (n=8)
G5: High fat group, culture liquid (stock) orally administered (n=8)

### EXAMPLE 11: Assay for Effect of Lactobacillus sakei CVL-001 Strain Isolated from Kimchi and Culture Liquid thereof (in vivo)

### 11-1. Change in body weight by Lactobacillus sakei CVL-001 strain or culture liquid thereof (in vivo)

Oral administration of a high fat diet including 60% of fats is known to further increase the weight of mice than that of a normal diet. Thus, high fat diet-induced obesity animal models are widely used for studies into obesity diseases.

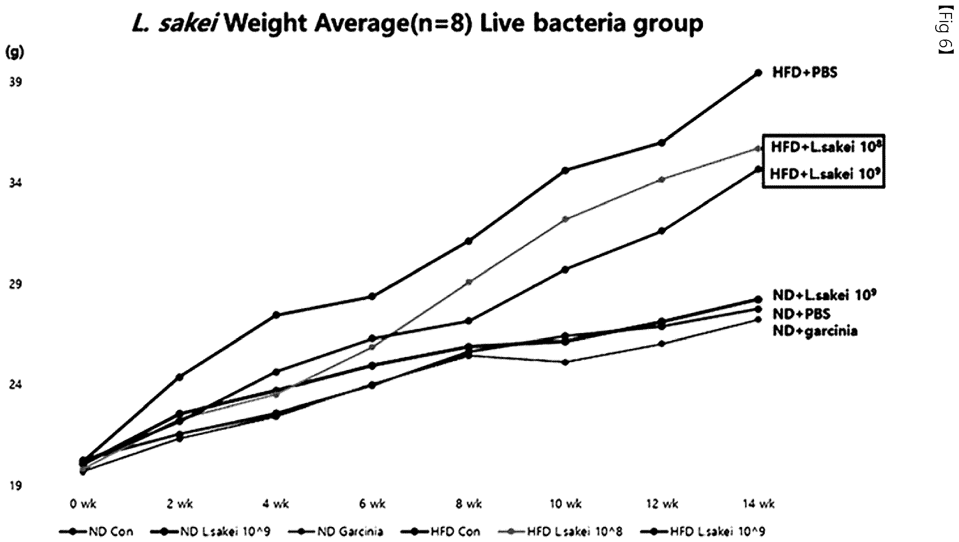

As can be seen in FIG. 6, significant weight gain was observed in the high fat diet-fed group (G3), compared to the normal diet-fed group (G1). Particularly, the groups (G4 and G5) to which the *Lactobacillus sakei* CVL-001 strain was fed at dose of 10⁸ and 10⁹ cells, respectively, together with the high fat diet were observed to significantly decrease in weight, compared to G3.

**[TABLE 6]**

| | G1 | G2 | G3 | G4 | G5 |
|---|---|---|---|---|---|
| Diet | Normal | Normal | High fat | High fat | High fat |
| Strain | PBS | *L. sakei* 10⁹ | PBS | *L. sakei* 10⁸ | *L. sakei* 10⁹ |
| Weight (g) | 0.80 | 0.85 | 2.29 | 2.23 | 1.93 |

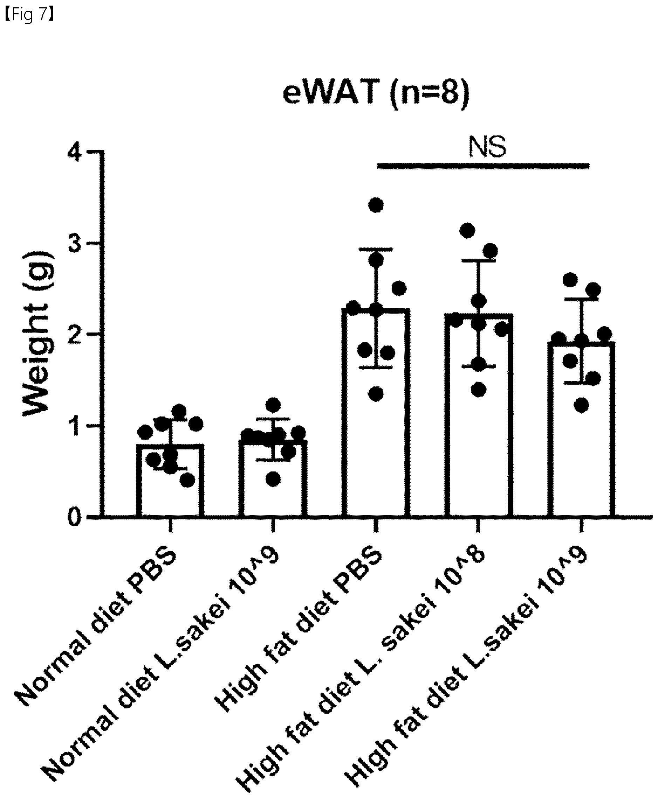

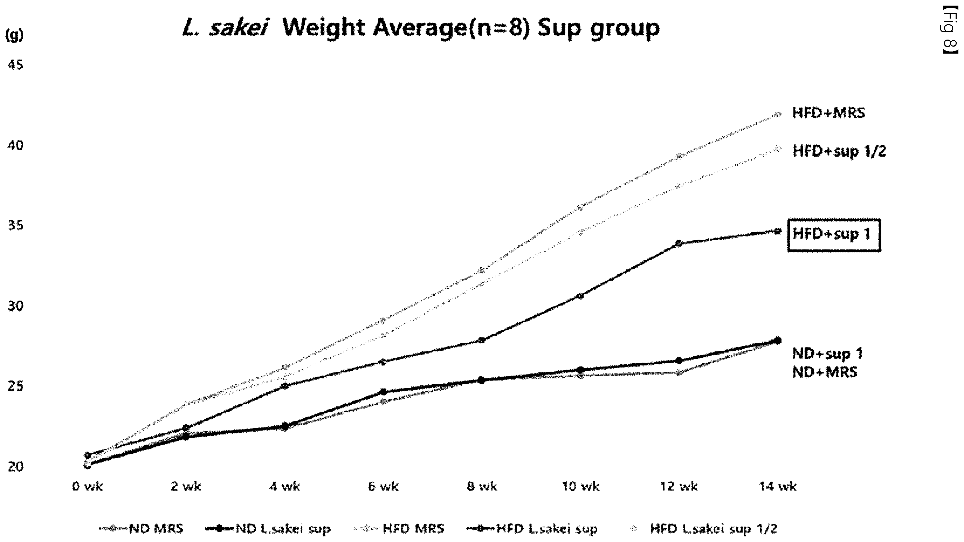

As shown in FIG. 7 and Table 6, G3 to which the high-fat was fed significantly increased in epididymal fat weight, compared to G1 while a significant reduction of epididymal fat weight was observed in G5 to which the strain had been fed at a dose of 10⁹ cells, together with a high fat diet. As shown in FIG. 8, the high fat diet-fed group (G3) was observed to significantly increase in body weight, compared to the normal diet-fed group (G1). Particularly, a significant reduction of body weight was detected in the group to which the *Lactobacillus sakei* CVL-001 culture liquid stock (G5) was fed, together with the high fat diet, compared to G3. The high fat diet-induced weight gain was not suppressed by administering the 1/2 diluted culture liquid (G4).

**[TABLE 7]**

| | G1 | G2 | G3 | G4 | G5 |
|---|---|---|---|---|---|
| Diet | Normal | Normal | High fat | High fat | High fat |
| Culture liquid | MRS | Culture liquid stock | MRS | Culture liquid 1/2 diluted | Culture liquid stock |
| Weight (g) | 0.744 | 0.691 | 2.579 | 2.065 | 1.768 |

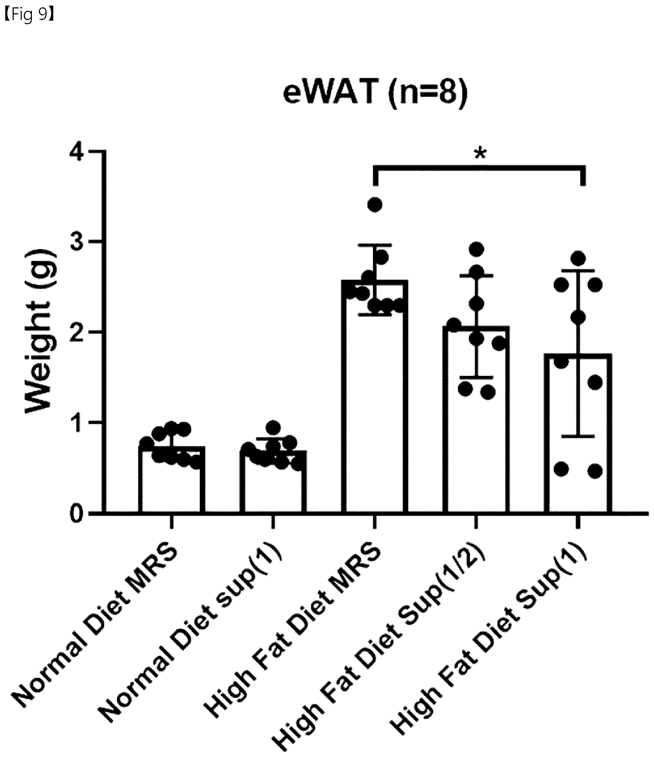

As is understood from data of FIG. 9 and Table 7, the epididymal fat weight was also significantly increased in the high fat diet-fed group G3 and significantly decreased in G5 to which the culture liquid stock had been fed, along with the high fat diet, compared to the normal diet fed group G1.

### 11-2. Fasting blood sugar test in obesity-induced mice after administration of Lactobacillus sakei CVL-001 strain and culture liquid

After being fasted for 12 hours from p.m. 9 before the day of experiment to a.m. 9 on the day of experiment, mice were subjected to a blood sugar test. The high fat-fed mouse animal models were measured to maintain high fasting blood sugar levels. Tests for experimental groups were conducted in the same conditions as in Example 10-3, and the groups to which the strain and the culture liquid were administered are listed in Tables 8 and 9, respectively.

**[TABLE 8]**

| Group | Fasting blood glucose (mg/dL) |
|---|---|
| G1: ND + control | 106.13±12.81^{a} |
| G2: ND + *L. sakei* 10^9 | 96.00±16.49^{a} |
| G3: HFD + control | 147.75±19.71^{b} |
| G4: HFD + *L. sakei* 10^9 | 117.63±25.04^{a} |
| G5: HFD + *L. sakei* 10^9 | 108.50±30.52^{a} |

As can be seen in Table 8, the high fat-fed group G3 maintained a significantly high fasting blood glucose level, compared to the normal diet-fed group G1 in this experiment. Particularly, blood glucose levels in G4 and G5 to which the *Lactobacillus sakei* CVL-001 strain had been administered at different doses, together with the high fat diet, were lower than that of G3 and similar to that of the normal diet-fed group G1.

**[TABLE 9]**

| Group | Fasting blood glucose (mg/dL) |
|---|---|
| G1: ND + MRS | 104.00±15.6^{a} |
| G2: ND + sup | 110.25±20.8^{a} |
| G3: HFD + MRS | 140.00±28.7^{b} |
| G4: HFD + sup(1/2) | 129.38±23.52^{b} |
| G5: HFD + sup | 106.75±15.23^{ab} |

As can be seen in Table 9, a significantly high fasting blood glucose level was maintained in the high fat diet-fed group G3, compared to the normal diet-fed group G1 in this experiment. Particularly, the blood glucose level in G5 to which the *Lactobacillus sakei* CVL-001 culture liquid stock had been fed, together with the high fat diet was observed to be low, compared to that of the culture liquid stock-administered group G3, and similar to that of the normal diet-fed group G1.

### 11-3. Glucose tolerance test in obesity-induced mice after administration of Lactobacillus sakei CVL-001 strain and culture liquid

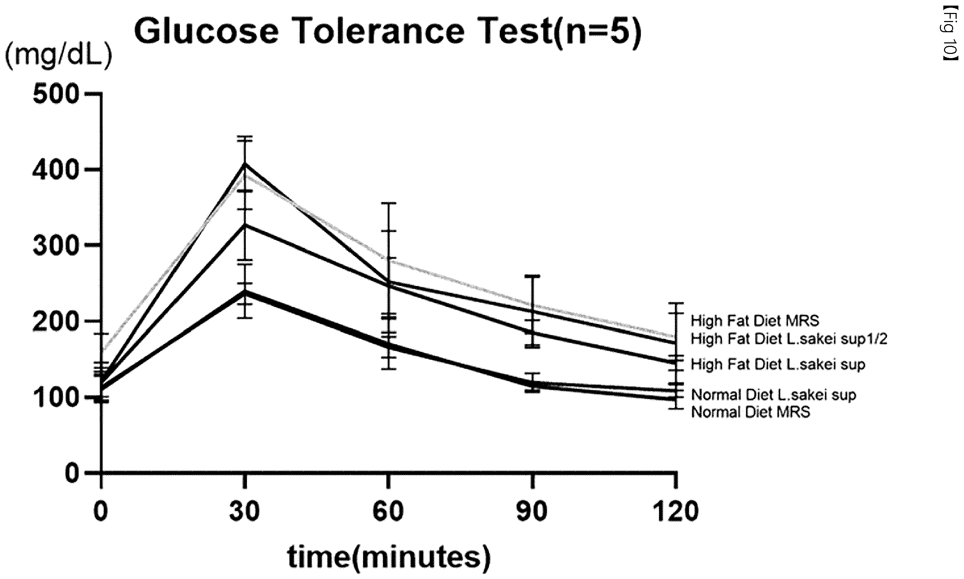

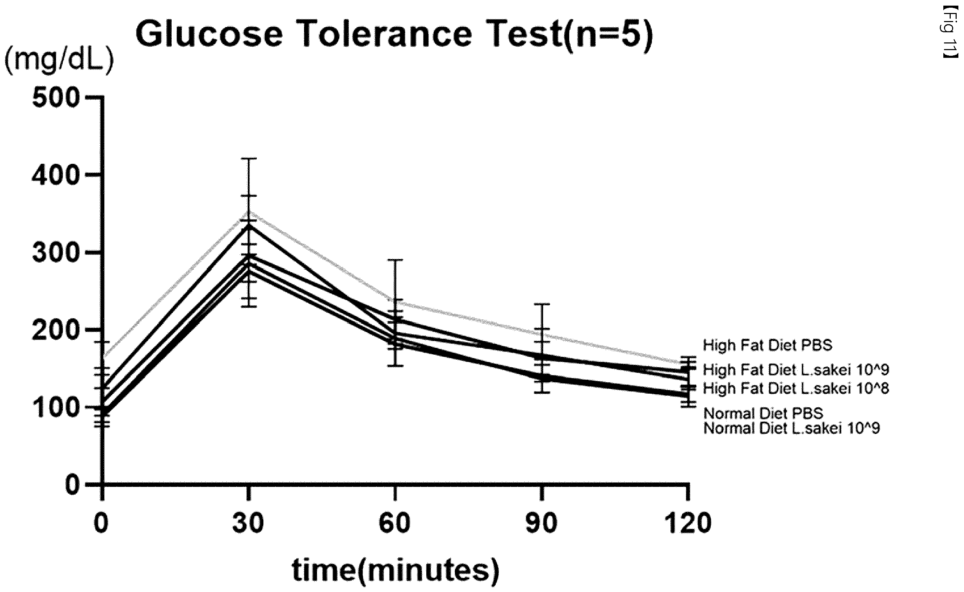

Tests for experimental groups were conducted in the same conditions as in Example 10-3, and mice were subjected to a glucose tolerance test after being fasted for 12 hours from p.m. 9 before the day of experiment to a.m. 9 on the day of experiment. A PBS solution containing 10% glucose, sterilized by 0.2 µm filtration) was intraperitoneally administered (27-gauge sterile needle) at a dose of 2 mg (glucose/g. volume (µl) = body weight (g) × 20) to each mouse. The mice were housed again in cages and measured for blood glucose level at time intervals of 0, 30, 60, 90, and 120 min. For the glucose test, blood was taken from tails. Measurements are given in Tables 10 and 11 and FIGS. 10 and 11.

**[TABLE 10]**

| Group | 0 | 30 | 60 | 90 | 120 (min) |
|---|---|---|---|---|---|
| Normal Diet MRS | 110.60± 17.52^{a} | 239.60± 35.70^{a} | 170.40± 32.99^{a} | 114.60± 5.75^{a} | 97.00± 12.38^{a} |
| Normal Diet sup | 113.20± 17.38^{a} | 236.40± 13.99^{a} | 166.00± 13.81^{a} | 119.40± 12.63^{a} | 108.40± 8.43^{ab} |
| High Fat Diet MRS | 159.00± 25.01b | 392.60± 45.12^{c} | 280.80± 75.26^{b} | 221.40± 37.10^{b} | 179.80± 30.93^{c} |
| High Fat Diet sup(1/2) | 121.20± 25.05^{a} | 407.20± 36.35^{c} | 252.40± 66.90^{b} | 213.20± 47.41^{b} | 171.60± 52.83^{c} |
| High Fat Diet sup | 119.80± 19.23^{a} | 327.00± 45.83^{b} | 246.80± 36.99^{b} | 185.20± 16.09^{b} | 145.40± 9.69^{bc} |

As shown in Table 10 and FIG. 10, the high fat diet-fed group (G3) was observed to maintain a significantly high blood sugar level, compared to the normal diet-fed group (G1). Particularly, a significant reduction of blood glucose level was detected in the group to which the *Lactobacillus sakei* CVL-001 culture liquid stock (G5) was administered, together with the high fat diet, compared to G3, from 30 min after administration. The high fat diet-induced blood glucose increase was not suppressed by administering the 1/2 diluted culture liquid (G4).

**[TABLE 11]**

| Group | 0 | 30 | 60 | 90 | 120 (min) |
|---|---|---|---|---|---|
| Normal Diet PBS | 89.00± 11.58^{a} | 261.80± 41.70^{a} | 175.40± 28.08^{a} | 136.60± 21.00^{a} | 114.40± 10.59^{a} |
| Normal Diet L. sakei 10^9 | 90.60± 10.15^{a} | 285.60± 55.98^{ab} | 188.80± 35.72^{ab} | 137.00± 17.94^{a} | 114.20± 14.01^{a} |
| High Fat Diet PBS | 163.20± 21.23^{c} | 352.60± 68.44^{b} | 236.00± 54.17^{b} | 194.00± 39.28^{b} | 155.40± 3.72^{b} |
| High Fat Diet L.sakei 10^8 | 124.00± 26.51^{b} | 335.00± 38.30^{ab} | 195.60± 20.55^{ab} | 167.20± 34.32^{ab} | 136.20± 13.42^{b} |
| High Fat Diet L. sakei 10^9 | 106.80± 18.15^{ab} | 296.00± 33.75^{ab} | 213.60± 25.42^{ab} | 163.60± 21.27^{ab} | 145.80± 19.20^{b} |

As can be seen in Table 11 and FIG. 11, a significant high level of blood glucose was observed in the high fat diet-fed group (G3), compared to the normal diet-fed group (G1). Particularly, the groups (G4 and G5) to which the *Lactobacillus sakei* CVL-001 strain was fed at dose of 10⁸ and 10⁹ cells, respectively, together with the high fat diet were observed to significantly decrease in blood glucose, compared to G3.

### 11-4. Insulin tolerance test in obesity-induced mice after administration of Lactobacillus sakei CVL-001 strain and culture liquid

Tests for experimental groups were conducted in the same conditions as in Example 10-3, and mice were subjected to an insulin tolerance test after being fasted for 4 hours from a.m. 9 to p.m. 1 on the day of experiment. To each mouse, 2 mg/ml insulin (in D.W. adjusted into a pH of 3 by diluted HCl) was intraperitoneally administered (27-gauge sterile needle) at a dose of 1 U (0.03846 mg/kg. Volume (ul) = Body weight (g) × 20). The mice were housed again in cages and measured for blood glucose level at time intervals of 0, 30, 60, 90, and 120 min. For the glucose test, blood was taken from tails.

**[TABLE 12]**

| Group | 0 | 30 | 60 | 90 | 120 (min) |
|---|---|---|---|---|---|
| Normal Diet MRS | 130.00± 10.06^{a} | 74.60± 9.77^{a} | 67.20± 15.45^{a} | 66.80± 14.19^{a} | 70.80± 8.30^{a} |
| Normal Diet *L. sakei* sup | 130.20± 6.58^{a} | 75.60± 11.48^{a} | 68.80± 24.21^{a} | 65.00± 19.83^{a} | 74.60± 19.30^{a} |
| High Fat Diet MRS | 179.40± 17.29^{b} | 119.80± 14.40^{b} | 117.00± 27.09^{b} | 145.40± 22.22^{b} | 183.60± 34.42^{c} |
| High Fat Diet *L. sakei* sup(1/2) | 180.40± 25.83^{b} | 114.80± 21.83^{b} | 95.60± 18.68^{ab} | 129.40± 62.94^{b} | 119.40± 38.71^{b} |
| High Fat Diet *L. sakei* sup | 156.60± 13.00^{b} | 110.20± 18.45^{b} | 89.00± 25.34^{ab} | 71.00+ 20.00^{a} | 83.40± 18.52^{ab} |

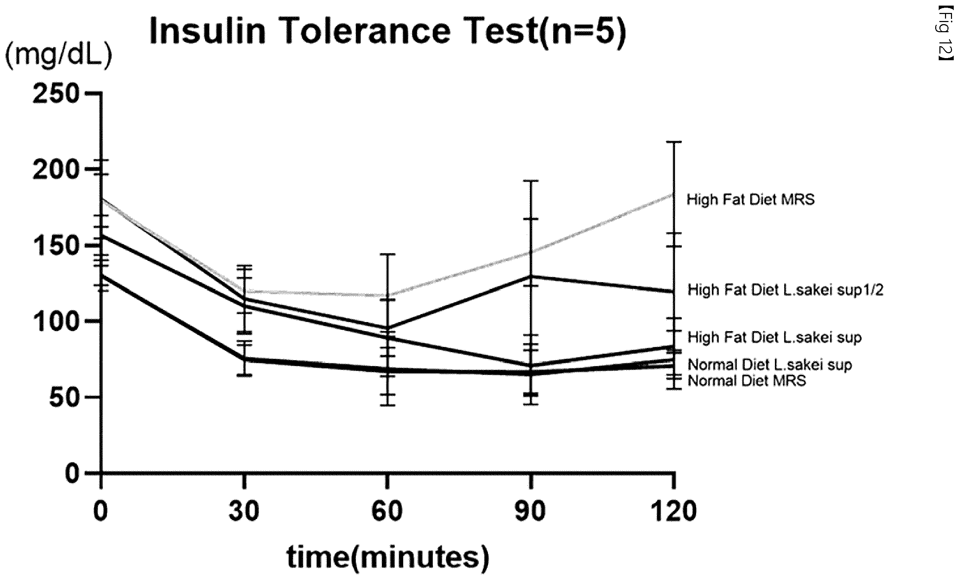

As shown in Table 12 and FIG. 12, the high fat diet-fed group (G3) was observed to maintain a significantly high blood sugar level, compared to the normal diet-fed group (G1). Particularly, a significant reduction of blood glucose level was detected in the group to which the *Lactobacillus sakei* CVL-001 culture liquid stock (G5) was administered, together with the high fat diet, compared to G3, at 90 and 120 min after administration. The group to which the 1/2 diluted culture liquid was administered (G4) exhibited a significantly decreased blood sugar level, compared G3, at 120 min after administration.

**[TABLE 13]**

| Group | 0 | 30 | 60 | 90 | 120 (min) |
|---|---|---|---|---|---|
| Normal Diet PBS | 140.40± 16.14^{a} | 96.60± 13.48^{ab} | 75.60± 8.69^{a} | 65.80± 7.76^{a} | 76.80± 10.91^{a} |
| Normal Diet *L*. *sakei* 10^9 | 126.40± 13.20^{a} | 77.20± 9.41^{a} | 69.00± 13.80^{a} | 56.20± 14.30^{a} | 66.20± 12.89^{a} |
| High Fat Diet PBS | 180.60± 22.66^{b} | 142.80± 31.04^{c} | 114.20± 16.08^{b} | 153.60± 37.32^{b} | 171.40± 23.14^{c} |
| High Fat Diet *L.sakei* 10^8 | 178.00± 12.54^{b} | 98.60± 10.95^{ab} | 81.20± 19.05^{a} | 83.20± 17.68^{a} | 115.80± 34.08^{b} |
| High Fat Diet *L*. *sakei* 10^9 | 177.20± 28.22^{b} | 121.00± 19.17^{bc} | 82.20± 23.13^{a} | 85.20± 13.12^{a} | 80.00± 18.48^{a} |

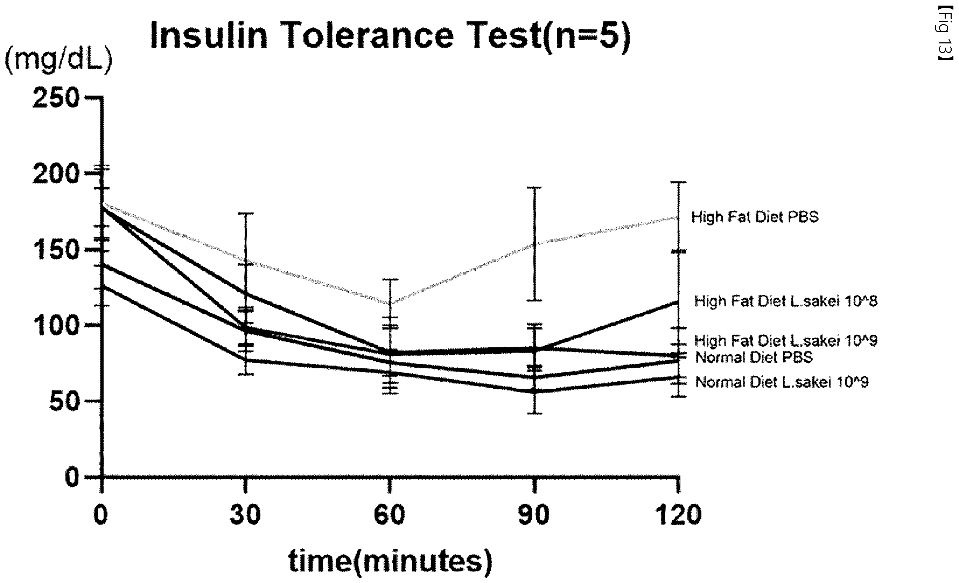

As can be seen in Table 13 and FIG. 13, a significant high level of blood glucose was observed in the high fat diet-fed group (G3), compared to the normal diet-fed group (G1). Particularly, the groups (G4 and G5) to which the *Lactobacillus sakei* CVL-001 strain was fed at dose of 10⁸ and 10⁹ cells, respectively, together with the high fat diet were observed to significantly decrease in blood glucose, compared to G3, at all times except for 0 min after administration.

### Industrial Applicability

The present disclosure relates to a *Lactobacillus sakei(Lactobacillus sakei*) CVL-001 strain and an isolation method therefor and, more specifically, to a *Lactobacillus sakei* CVL-001 strain isolated and identified from kimchi.

In addition, the present disclosure relates to a composition comprising the *Lactobacillus sakei* CVL-001 strain or a culture liquid thereof for alleviation, prevention, or treatment of a bone disease and, more specifically, to a composition comprising a *Lactobacillus sakei* CVL-001 strain deposited under accession number KCTC13816BP or a culture liquid thereof for alleviation, prevention, or treatment of a bone disease.

INTERNATIONAL FORM
RECEIPT IN THE CASE OF AN ORIGINAL DEPOSIT
issued pursuant to Rule 7.1

TO: Industry Foundation of Chonnam National University
Chonnam National University
77, Yongbong-ro, Buk-gu, Gwangju 61186
Republic of Korea

I. IDENTIFICATION OF THE MICROORGANISM
Identification reference given by the
DEPOSITOR:

Lactobacillus sakei CVL-001 | Accession number given by the
INTERNATIONAL DEPOSITARY AUTHORITY:

KCTC 13816BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on February 19, 2019.
IV. RECEIPT OF REQUEST FOR CONVERSION
The microorganism identified under I above was received by this International Depositary Authority on
and a request to convert the original deposit to a deposit under the
Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeolllabuk-do 56212
Republic of Korea | Signature(s) of person(s) having the power to represent the
International Depositary Authority or of authorized
official(s):

Song-Gun Kim

KIM, Song-Gun, Director
Date: February 19, 2019
Form BP/4 (KCTC Form 17) sole page

<110>    Industry-University Cooperation Foundation Chonnam University

<120>    COMPOSITION COMPRISING LACTOBACILLUS SAKEI CVL-001 OR CULTURE
         LIQUID THEREOF FOR ALLEVIATING, PREVENTING, OR TREATING BONE
         DISEASES OR METABOLIC DISEASES

<130>    PP200015EP

<150>    KR 10-2019-0022670
<151>    2019-02-26

<150>    KR 10-2019-0022671
<151>    2019-02-26

<150>    KR 10-2020-0012763
<151>    2020-02-03

<160>    3

<170>    KoPatentIn 3.0

<210>    1
<211>    1439
<212>    DNA
<213>    Unknown

<220>
<223>    Lactobacillus sakei CVL-001 16S rRNA


<400>    1
gcagtcgaac gcactctcgt ttagattgaa ggagcttgct cctgattgat aaacatttga          60

gtgagtggcg gacgggtgag taacacgtgg gtaacctgcc ctaaagtggg ggataacatt         120

tggaaacaga tgctaatacc gcataaaacc taacaccgca tggtgtaggg ttgaaagatg         180

gtttcggcta tcactttagg atggacccgc ggtgcattag ttagttggtg aggtaaaggc         240

tcaccaagac cgtgatgcat agccgacctg agagggtaat cggccacact gggactgaga         300

cacggcccag actcctacgg gaggcagcag tagggaatct tccacaatgg acgaaagtct         360

gatggagcaa cgccgcgtga gtgaagaagg ttttcggatc gtaaaactct gttgttggag         420

aagaatgtat ctgatagtaa ctgatcaggt agtgacggta tccaaccaga aagccacggc         480

taactacgtg ccagcagccg cggtaatacg taggtggcaa gcgttgtccg gatttattgg         540

gcgtaaagcg agcgcaggcg gtttcttaag tctgatgtga aagccttcgg ctcaaccgaa         600

gaagtgcatc ggaaactggg aaacttgagt gcagaagagg acagtggaac tccatgtgta         660

gcggtgaaat gcgtagatat atggaagaac accagtggcg aaggcggctg tctggtctgt         720

aactgacgct gaggctcgaa agcatgggta gcaaacagga ttagataccc tggtagtcca         780

tgccgtaaac gatgagtgct aggtgttgga gggtttccgc ccttcagtgc cgcagctaac         840

gcattaagca ctccgcctgg ggagtacgac cgcaaggttg aaactcaaag gaattgacgg         900

gggcccgcac aagcggtgga gcatgtggtt taattcgaag caacgcgaag aaccttacca         960

ggtcttgaca tcctttgacc actctagaga tagagctttc ccttcgggga caaagtgaca 1020

ggtggtgcat ggttgtcgtc agctcgtgtc gtgagatgtt gggttaagtc ccgcaacgag 1080

cgcaaccctt attactagtt gccagcattt agttgggcac tctagtgaga ctgccggtga 1140

caaaccggag gaaggtgggg acgacgtcaa atcatcatgc cccttatgac ctgggctaca 1200

cacgtgctac aatggatggt acaacgagtt gcgagaccgc gaggtttagc taatctctta 1260

aaaccattct cagttcggat tgtaggctgc aactcgccta catgaagccg gaatcgctag 1320

taatcgcgga tcagcatgcc gcggtgaata cgttcccggg ccttgtacac accgcccgtc 1380

acaccatgag agtttgtaac acccaaagcc ggtgaggtaa cccttcgggg agccagccg 1439

<210> 2
<211> 20
<212> DNA
<213> Artificial Sequence

<220>
<223> Lactobacillus sakei 16S rRNA forward primer

<400> 2
agagtttgat cctggctcag 20

<210> 3
<211> 19
<212> DNA
<213> Artificial Sequence

<220>
<223> Lactobacillus sakei 16S rRNA reverse primer

<400> 3
ggttaccttg ttacgactt 19

## Claims

1. A *Lactobacillus sakei* CVL-001 strain, deposited under accession number KCTC13816BP.

2. The *Lactobacillus sakei* CVL-001 strain of claim 1, wherein the strain includes a 16S rRNA sequence represented by SEQ ID NO: 1.

3. A culture liquid of a *Lactobacillus sakei* CVL-001 strain deposited under accession number KCTC13816BP.

4. The culture liquid of claim 3, wherein the strain includes a 16S rRNA sequence represented by SEQ ID NO: 1.

5. A method for isolating a *Lactobacillus sakei* CVL-001 strain deposited under accession number KCTC13816BP, the method comprising a step of culturing a kimchi extract.

6. The method of claim 5, wherein the kimchi extract is a kimchi-ground juice obtained by grinding kimchi and filtering the ground kimchi.

7. The method of claim 5, wherein the culturing step comprises a first culturing step for incubating the kimchi extract; and a second culturing step for culturing a bacterial strain isolated in the first culturing step.

8. The method of claim 7, wherein the first culturing step is conducted in a medium containing calcium carbonate.

9. The method of claim 7, wherein the first culturing step is conducted at 20 to 32°C for 36 to 60 hours.

10. The method of claim 7, wherein the second culturing step is a step in which selection is made of a Gram-positive lactic acid bacterium from the isolated strains.

11. The method of claim 7, wherein the second culturing step is a step in which selection is made of a catalase-negative lactic acid bacterium from the isolated strains.

12. The method of claim 7, wherein the second culturing step is conducted at 20 to 32°C for 12 to 36 hours.

13. A pharmaceutical composition comprising a *Lactobacillus sakei* CVL-001 strain deposited under accession number KCTC13816BP, or a culture liquid thereof for preventing or treating a bone disease or a metabolic disease.

14. The pharmaceutical composition of claim 13, wherein the bone disease is selected from the group consisting of osteoporosis, bone metastatic cancer, osteomalacia, rickets, fibrous osteitis, adynamic bone disease, metabolic disease, and periodontal disease.

15. The pharmaceutical composition of claim 13, wherein the metabolic disease is at least one selected from the group consisting of obesity, diabetes mellitus, hypertension, hyperlipidemia, cardiovascular disease, and hyperinsulinemia.

16. The pharmaceutical composition of claim 13, wherein the culture liquid is a supernatant obtained by culturing the *Lactobacillus sakei* CVL-001 strain and removing the same, a concentrate thereof, a fraction thereof, or a lyophilisate thereof.

17. The pharmaceutical composition of claim 13, wherein the pharmaceutical composition contains the *Lactobacillus sakei* CVL-001 strain at a concentration of 5 × 10⁵ to 5 × 10¹² CFU/ml.

18. The pharmaceutical composition of claim 13, wherein the culture liquid is obtained by culturing the *Lactobacillus sakei* CVL-001 strain for 12 to 30 hours.

19. The pharmaceutical composition of claim 13, wherein the composition contains the culture liquid at a concentration of 50 to 400 µl/ml.

20. The pharmaceutical composition of claim 13, wherein the culture liquid has a pH of 6.5 to 8.5.

21. A food composition comprising a *Lactobacillus sakei* CVL-001 strain deposited under accession number KCTC13816BP, or a culture liquid thereof for alleviating a bone disease or a metabolic disease.

22. The food composition of claim 21, wherein the bone disease is selected from the group consisting of osteoporosis, bone metastatic cancer, osteomalacia, rickets, fibrous osteitis, adynamic bone disease, metabolic disease, and periodontal disease.

23. The food composition of claim 21, wherein the metabolic disease is at least one selected from the group consisting of obesity, diabetes mellitus, hypertension, hyperlipidemia, cardiovascular disease, and hyperinsulinemia.

24. The food composition of claim 21, wherein the culture liquid is a supernatant obtained by culturing the *Lactobacillus sakei* CVL-001 strain and removing the same, a concentrate thereof, a fraction thereof, or a lyophilisate thereof.

25. The food composition of claim 21, wherein the culture liquid is obtained by culturing the *Lactobacillus sakei* CVL-001 strain for 12 to 30 hours.

26. The food composition of claim 21, wherein the food composition contains the *Lactobacillus sakei* CVL-001 strain at a concentration of 5 × 10⁵ to 5 × 10¹² CFU/ml.

27. The food composition of claim 21, wherein the composition contains the culture liquid at a concentration of 50 to 400 µl/ml.

28. The food composition of claim 21, wherein the culture liquid has a pH of 6.5 to 8.5.
